# EUROPEAN PATENT SPECIFICATION

(11) **EP 1 478 636 B1**
(45) Date of publication and mention of the grant of the patent: **19.11.2008**
(21) Application number: 03704820.4
(22) Date of filing: 26.02.2003
(51) Int. Cl.: C07D 307/87, A61K 31/343, A61P 25/00

(54) **AMORPHOUS CITALOPRAM**
AMORPHES CITALOPRAM
CITALOPRAM AMORPHE

(30) Priority: 27.02.2002 GB 0204680
(43) Date of publication of application: 24.11.2004
(73) Proprietor: Cipla Ltd., Mumbai 400 008 (IN)
(72) Inventor: HAMIED, Yusuf K., Windsor Villa, 2nd floor, Mumbai 400 026 (IN); N KANKAN, Rajendra, A-3/5 NBD Society, Mumbai 400 084, Maharashtra (IN); R RAO, Dharmaraj, Thane 400 601, Maharashtra (IN)
(74) Representative: Wain, Christopher Paul
(86) International application number: PCT/GB2003/000810
(87) International publication number: WO 2003/072562

(56) References cited:
- WO-A-01/68627
- GB-A- 2 357 762
- CHEMICAL ABSTRACTS, vol. 126, no. 3, 20 January 1997 (1997-01-20) Columbus, Ohio, US; abstract no. 26304, HAUPT D.: "Determination of citalopram enantiomers in human plasma by liquid chromatographic separation on a chiral-AGP column" XP002225392 & J. CHROMATOGR., B: BIOMED. APPL., vol. 685, no. 2, 1996, pages 299-305,
- CHEMICAL ABSTRACTS, vol. 105, no. 10, 8 September 1986 (1986-09-08) Columbus, Ohio, US; abstract no. 85185, FUKUYAMA Y. ET AL: "Phtalimides from ligusticum wallichi" XP002225344 & JP 61 007267 A (OTSUKA PHARM. CO., LTD.) 13 January 1986 (1986-01-13)

## Description

This invention relates to a pharmaceutical product, more particularly to citalopram.

Citalopram is a well known antidepressant drug whose systematic name is 1-[3-(dimethylamino)propyl]-1-(4-fluorophenyl)-1,3-hydro-5-isobenzofurancarbonitrile. It is a selective centrally acting serotonin (S-hydroxytryptamine; 5-HT) reuptake inhibitor. It is marketed as the hydrobromide or hydrochloride salt.

Citalopram was first described in GB-A-1526331 and, subsequently, a number of different processes have been described for its preparation. In many of these, the final step is to introduce the 5-cyano group but there have been problems in purifying the final product to remove intermediates and by-products. Among the purification processes used has been isolation of the free base as an oil (bp 175°C/0.03 mm Hg) and subsequent thin film distillation followed by conversion to the desired salt. Another purification process involves conversion to a salt and recrystallisation thereof Neither of these techniques has been particularly satisfactory.

Recently, another purification procedure has been described in GB-B-2357762. Here, citalopram base is set free and precipitated in crystalline form, and after optional recrystallisation for purification, converted to the desired salt. This process is said to be particularly effective at removed 5-substituted intermediate contaminants. A disadvantage of this is that it requires repeated crystallisations to achieve high purity and this is undesirable.

We have now found another way of purifying citalopram which has a number of advantages over prior known processes.

Further, we have found that a previously undescribed form of citalopram base, namely amorphous citalopram base, has excellent utility. Thus, the invention provides amorphous citalopram base *per se*, including amorphous S-citalopram base.

According to the invention, citalopram purified by chromatography, is converted to the amorphous base and, optionally, as desired, to the desired salt.

In the process of the present invention, citalopram is separated from (ie isolated from) impurities, preferably on a single column without multiple elutions. This is a commercial scale operation resulting in purified citalopram. This is quite different from for example, laboratory techniques for determining citalopram and its metabolites in plasma, which types of procedure use an analytical HPLC column and are not a preparatory method, and do not result in isolation of the product but rather its estimation in solution.

The crude citalopram which can be purified by chromatography in accordance with the present invention may have been made in any way such as, for example, by any of the methods known in the art. The purification method is particularly useful with crude citalopram made by a step including exchange of a 5-halo substituent by a 5-cyano substituent using, for example, sodium, potassium, cuprous or zinc cyanide, with or without catalysts or solvents.

Any suitable form of chromatography can be used including, for example, medium pressure liquid chromatography (MPLC), high performance liquid chromatography (HPLC), or simulated moving bed chromatography (SMB).

Thus, for example, crude citalopram may be loaded on MPLC with a suitable stationary phase for normal or reverse phase chromatography. The product from this purification procedure is substantially free of all the known impurities of citalopram such as the 5-carboxamide impurity, 5-chloro impurity, 5-bromo impurity and the N-desmethyl impurity. Typically the level of each of these impurities in the purified citalopram is less than 0.1%.

Alternatively, preparative HPLC can be used, employing a suitable stationary phase and mobile phase, or SMB can be used with a suitable stationary phase and mobile phase. These techniques are well known in the art and further description thereof will not therefore be given.

The purified citalopram base resulting from the chromatography will be in solution in the eluant and is recovered as the amorphous base. This can be effected in a number of ways such as by lyophilisation or evaporation (eg by a rotary evaporation), as will be clear to those skilled in the art. We prefer, however, to use spray drying to obtain the amorphous citalopram base.

Salts of the purified base can be made by routine procedures either directly from the eluate solution, or from the amorphous base, or by any other route as desired. The preferred salts are the hydrobromide, hydrochloride and oxalate, but others can of course be made.

In order that the invention may be more fully understood, the following Examples are given by way of illustration only.

### Example 1

Crude citalopram base 10 g is dissolved in 20 ml dichloromethane and loaded on a column containing silica gel 60 - 120# (300 g) and eluted with a gradient mixture of dichloromethane, toluene and methanol. The eluent was monitored by HPLC and the fractions containing pure citalopram were concentrated to obtain an oil. Purity of the oil was 99.5%

### Example 2

Crude citalopram base 10 g is dissolved in 20 ml toluene and loaded on a column containing neutral alumina (300 g) and eluted with a gradient mixture of toluene and dichloromethane. The eluent was monitored by HPLC and the fractions containing pure citalopram were concentrated to obtain an oil. Purity of the oil was 99.5%

### Example 3

Crude citalopram base 20 g is dissolved in 20 ml dichloromethane and loaded on a MPLC column 90 mm x 1500 mm containing silica gel 30-40 microns and eluted with a gradient mixture of dichloromethane toluene and methanol. The fractions containing pure citalopram were concentrated to obtain an oil. Purity of the oil was 99.5%.

### Example 4

Crude citalopram base 20 g is dissolved in 20 ml acetonitrile and loaded on a MPLC column 90 mm x 1500 mm packed with RP 18, 40 - 60 micron and eluted with a gradient mixture of acetonitrile and water. The fractions containing pure citalopram were concentrated to obtain an oil. Purity of the oil was 99.5%.

### Example 5

The oil from Example 1 was dissolved in methanol to obtain a 10% solution. This was spray dried on a Lab-Plant spray drier SD-05 with an inlet temperature of 80°C and outlet temperature of 45°C at a feed rate of about 10ml/min to obtain a fine amorphous solid which was characterised by powder x-ray diffraction.

### Example 6

The oil obtained from Example 2 was dissolved in ethyl acetate (5v/w) and treated with aqueous hydrobromic acid 48% to a pH of about 3.5. The solids were filtered and dried to obtain citalopram hydrobromide.

## Claims

1. Amorphous citalopram base.

2. A method of preparing amorphous citalopram base, which comprises subjected a solution of citalopram base to spray drying, lyophilisation or evaporation, and recovering amorphous citalopram base, wherein the solution of citalopram base is obtained by purifying citalopram base by subjecting it to chromatography.

3. A method according to claim 2, wherein the citalopram base is purified by liquid chromatography.

4. A method according to claim 3, wherein the base is purified by medium or high pressure liquid chromatography or by simulated moving bed chromatography.

5. A method of making a salt of citalopram which comprises converting amorphous citalopram base into a salt.

6. A method according to claim 5, wherein the amorphous base has been made by the method of claim 2, 3 or 4.

7. A pharmaceutical composition which comprises amorphous citalopram base.

## Patentansprüche

1. Amorphe Citaloprambase.

2. Verfahren zum Herstellen von amorpher Citaloprambase, welches das Unterwerfen einer Lösung von Citaloprambase einer Sprühtrocknung, Lyophilisierung oder Eindampfung und das Gewinnen von amorpher Citaloprambase umfasst, wobei die Lösung von Citaloprambase durch das Reinigen von Citaloprambase durch das Unterwerfen der Citaloprambase einer Chromatographie erhalten wird.

3. Verfahren nach Anspruch 2, wobei die Citaloprambase durch Flüssigchromatografie gereinigt wird.

4. Verfahren nach Anspruch 3, wobei die Base durch Mittel- oder Hochdruckflüssigchromatografie oder durch simulierte Wanderbettchromatografie gereinigt wird.

5. Verfahren zum Herstellen eines Salzes von Citalopram, welches das Umwandeln von amorpher Citaloprambase in ein Salz umfasst.

6. Verfahren nach Anspruch 5, wobei die amorphe Base durch das Verfahren von Anspruch 2, 3 oder 4 hergestellt worden ist.

7. Pharmazeutische Zusammensetzung, welche amorphe Citaloprambase umfasst.

## Revendications

1. Base de citalopram amorphe.

2. Procédé pour la préparation d'une base de citalopram amorphe qui comprend le fait de soumettre une solution de base de citalopram à un séchage par atomisation, lyophilisation ou évaporation et le fait de récupérer la base de citalopram amorphe, dans lequel la solution de la base de citalopram est obtenue en purifiant une base de citalopram en la soumettant à une chromatographie.

3. Procédé selon la revendication 2, dans lequel la base de citalopram est purifiée par chromatographie en phase liquide.

4. Procédé selon la revendication 3, dans lequel la base est purifiée par une chromatographie en phase liquide à pression moyenne ou élevée ou par chromatographie à lit mobile simulé.

5. Procédé pour la fabrication d'un sel de citalopram qui comprend la conversion d'une base de citalopram amorphe en un sel.

6. Procédé selon la revendication 5, dans lequel la base amorphe a été fabriquée par le procédé des revendications 2, 3 ou 4.

7. Composition pharmaceutique qui comprend une base de citalopram amorphe.
